(19) Europäisches Patentamt
European Patent Office
Office européen des brevets

(11) Veröffentlichungsnummer: **0 316 791 B1**

(12) **EUROPÄISCHE PATENTSCHRIFT**

(45) Veröffentlichungstag der Patentschrift: **10.03.93**

(51) Int. Cl.$^5$: **C07F 9/12**, C12Q 1/42

(21) Anmeldenummer: **88118800.7**

(22) Anmeldetag: **11.11.88**

# (54) 1-Naphtholphthaleinmonophosphate, Verfahren zu deren Herstellung sowie deren Verwendung zur Bestimmung der alkalischen Phosphatase.

(30) Priorität: **20.11.87 DE 3739284**

(43) Veröffentlichungstag der Anmeldung:
**24.05.89 Patentblatt 89/21**

(45) Bekanntmachung des Hinweises auf die Patenterteilung:
**10.03.93 Patentblatt 93/10**

(84) Benannte Vertragsstaaten:
**AT BE CH DE ES FR GB GR IT LI LU NL SE**

(56) Entgegenhaltungen:
**US-A- 3 975 405**

(73) Patentinhaber: **BOEHRINGER MANNHEIM GMBH**
**Sandhofer Strasse 116**
**W-6800 Mannheim 31(DE)**

(72) Erfinder: **Güthlein, Werner, Dr. rer. nat.**
**Im Sennteich 31**
**W-6800 Mannheim 24(DE)**
Erfinder: **Merdes, Hartmut, Dr. rer. nat.**
**Schusterstrasse 11**
**W-6900 Heidelberg 1(DE)**

**EP 0 316 791 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue Phosphorsäuremonoester sowie deren Salze, die als Enzymsubstrate unter dem Einfluß von Esterasen bei alkalischen pH-Werten, speziell von alkalischer Phosphatase (EC 3.1.3.1), ein farbiges Anion bilden sowie die Herstellung dieser Phosphorsäureester bzw. entsprechender Salze und ein Verfahren und Mittel zum Nachweis und zur photometrischen Bestimmung der alkalischen Phosphatase.

Alkalische Phosphatase (AP) kommt beispielsweise in der Leber, in den Knochen, in dem Dünndarm, in den Nieren, in der Galle und in geringerem Ausmaß in der Placenta vor. Der Anstieg der AP-Aktivität im Plasma hat insbesondere für den Nachweis von Lebererkrankungen und Erkrankungen des Knochenskeletts diagnostische Bedeutung. Beispielsweise werden erhöhte AP-Spiegel bei Morbus Paget, bei Osteosarkomen, Gelbsucht, Hepatitis und ähnlichen Krankheitsbildern beobachtet.

Über ihren physiologischen Stellenwert hinaus hat die alkalische Phosphatase in den letzten Jahren als diagnostisches Hilfsmittel an Bedeutung gewonnen. So wird beispielsweise dieses Enzym als Indikator-Enzym für Enzymimmunoassays eingesetzt.

Die klinische Chemie sowie die Diagnostik erfordern somit eine einfach zu handhabende und zugleich gut reproduzierbare Methode zur quantitativen Bestimmung der alkalischen Phosphatase. Diesem Zweck dient in der Regel die direkte photometrische oder fluorometrische Bestimmung von Verbindungen, die durch AP bewirkte hydrolytische Spaltung aus zugesetzten spezifischen Substraten hervorgegangen sind. Bei den durch alkalische Phosphatase freigesetzten Verbindungen handelt es sich um chromogene bzw. mesomeriestabilisierte Alkohole, insbesondere Phenolderivate, und Phosphatreste. In der Regel werden erstere qualitativ bzw. quantitativ bestimmt.

Sowohl bei colorimetrischen sowie bei fluorometrischen Bestimmungen ist die parallele oder nachgeschaltete Durchführung eines Leerwertes (ohne Probe, d. h. ohne AP) ratsam, um Interferenzen durch eine eventuell vorhandene Absorption des nicht umgesetzten Substrats erkennen zu können.

Neben p-Nitrophenylphosphat, Naphthylphosphaten und Phenolphthaleindiphosphaten bzw. entsprechender Derivate sind Phenolphthaleinmonophosphorsäure bzw. deren Salze als AP-Substrate geeignet (US-Patente 3.331.857 und US 3.331.862). Diese Verbindungen sind jedoch vor allem insofern für die Bestimmung der alkalischen Phosphatase unbefriedigend, als der Nachweis der freigesetzten Phenolphthaleinmenge durch andere in der Probe vorhandene Verbindungen verfälscht wird. Denn zahlreiche Serumbestandteile, wie beispielsweise Bilirubin haben wie die erwähnten aus den AP-Substraten freigesetzten Phenolate ein Absorptionsmaximum bei ca. 400 - 450 nm.

Bevorzugt werden daher heute Thymolphthaleinmonophosphorsäure bzw. wegen der guten Wasserlöslichkeit die entsprechenden Natrium- und Ammoniumsalze als Substrate zur Bestimmung der alkalischen Phosphatase verwendet (Clin. Chem. 16, 431 - 436 (1970); Clin. Chem. 19, 1135 - 1138 (1973); JP 83 158 199 (1983) ). Freigesetztes Thymolphthalein zeigt eine maximale Absorptionsbande bei ca. 595 nm im Absorptions- bzw. Emissionsspektrum. Nachteilig bei diesen Substanzen ist jedoch zum einen die ungenügende Empfindlichkeit bei Intestinal- und Placenta-AP, zum anderen der Umstand, daß keine kinetische Bestimmung, sondern stattdessen eine Endpunktsbestimmung durchgeführt werden muß, die einen Umpufferungsschritt beinhaltet.

o-Kresolphthaleinmonophosphorsäure ist ein weiteres Phenolphthaleinderivat, das heute üblicherweise als AP-Substrat eingesetzt wird (US-Patent 3.975.405). Unter Zuhilfenahme dieses Substrats ist sowohl die kontinuierliche als auch die nicht-kontinuierliche Bestimmung der alkalischen Phosphatase möglich, da das freigesetzte o-Kresolphthalein im Gegensatz zu Thymolphthalein schon in einem für die enzymatische AP-Bestimmung optimalen pH-Bereich absorbiert. Jedoch wird die spektroskopische Bestimmung des freigesetzten o-Kresolphthaleins in Gegenwart hämolytischer Proben gestört, da das Absorptionsmaximum des o-Kresolphthaleinanions bei ca. 570 nm liegt.

Aufgabe der Erfindung war es somit, neue Substrate für die Bestimmung der alkalischen Phosphatase zur Verfügung zu stellen, die neben ausreichender Wasserlöslichkeit die Eigenschaft besitzen, unter dem Einfluß des Enzyms Hydrolyseprodukte freizusetzen, die sich durch möglichst langwellige Absorptionsmaxima auszeichnen und somit bei der photometrischen Bestimmung durch andere Probenbestandteile wie insbesondere Hämolyseprodukte nicht oder nur geringfügig beeinflußt werden. Außerdem sollten die neuen AP-Substrate ausreichend stabil sein und durch einfache und wirtschaftlich interessante Synthese in hoher Reinheit zugänglich sein. Gelöst wird diese Aufgabe durch die erfindungsgemäßen 1-Naphtholphthaleinmonophosphate, die unter dem Einfluß von alkalischen Esterasen, insbesondere von alkalischen Phosphatasen, zu langwellig absorbierenden Anionen umgesetzt werden. Die Erfindung betrifft Verbindungen der allgemeinen Formel I

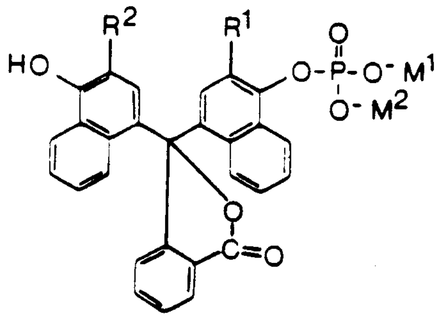

(I)

in der
$R^1$ und $R^2$, die gleich oder verschieden sein können, jeweils Wasserstoff oder Halogen
$M^1$ und $M^2$, die gleich oder verschieden sein können, jeweils ein Proton, ein Alkalimetall-, ein Erdalkalimetall-, ein Ammonium-, ein Dicyclohexylammonium-, ein Tetraalkylammonium- oder ein Alkanolammoniumkation bedeuten, und ihre tautomeren Umwandlungsprodukte.

Sämtliche 1-Naphtholphthaleinmonophosphate der allgemeinen Formel I sind neue Verbindungen. Sie können nach an sich aus der Phenolchemie her bekannten Methoden hergestellt werden.

Vorzugsweise werden in an sich bekannter Weise 1-Naphtholphthalein bzw. dessen Salze der allgemeinen Formel II

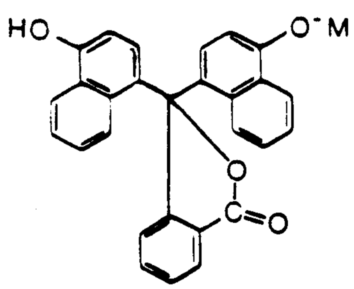

(II)

in der M ein Proton, ein Alkalimetall-, ein Erdalkalimetall-, ein Ammonium-, ein Dicyclohexylammonium-, ein Tetraalkylammonium- oder ein Alkanolammoniumkation bedeutet, mit einer Phosphorverbindung der allgemeinen Formel III

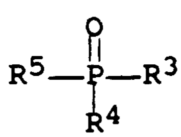

(III)

in der $R^3$ und $R^4$, die gleich oder verschieden sein können, jeweils Halogen, eine Aryl- oder eine Alkylarylgruppe und $R^5$ Wasserstoff oder Halogen bedeuten, in Gegenwart einer organischen Base in wasserfreiem Lösungsmittel bzw. Lösungsmittelgemisch umsetzt und gegebenenfalls bromiert.

Beispielsweise können 1-Naphtholphthaleinmonophosphate der allgemeinen Formel I, in der $R^1$ und $R^2$ Wasserstoff bedeuten und $M^1$ und $M^2$ die oben angegebene Bedeutung haben, mit der äquimolaren Menge Phosphoroxichlorid in Gegenwart von 0,93 Mol Pyridin umgesetzt werden. Dabei entsteht im wesentlichen das gewünschte Monophosphat I, worin $R^1$ und $R^2$ Wasserstoff bedeuten, neben wenig Diphosphat und Ausgangsmaterial, das durch Ausschütteln mit Butanol/Ligroin = 1/1 bei pH 10,3 entfernt wird. Die Abtrennung des Monophosphats vom Diphosphat wurde durch Säulenchromatographie erreicht.

Des weiteren können die Monophosphate der allgemeinen Formel I analog der Synthese von o-Kresolphthaleinmonophosphat nach Coulter (US-Patent 3975405) hergestellt werden, indem man 1-Naphtholphthalein der allgemeinen Formel II und Dibenzylphosphit mit Tetrachlorkohlenstoff in absolutem Tetrahydrofuran in Gegenwart einer organischen Base, beispielweise Triethylamin, zu Dibenzylphosphorsäureester der Formel IV

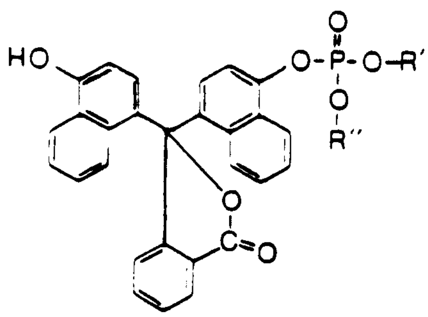

(IV)

in der R′ und R″ jeweils Benzylgruppen bedeuten, umsetzt. Verbindungen der Formel IV werden durch Säulenchromatographie gereinigt und durch katalytisch aktivierten Wasserstoff in Gegenwart von Palladium/Kohlenstoff debenzyliert. Nach weiterer säulenchromatographischer Reinigung erhält man Verbindungen der allgemeinen Formel I, worin $R^1$ und $R^2$ Wasserstoff bedeuten.

Anstatt von Dibenzylphosphit kann außerdem Diphenylphosphit verwendet werden. Es entstehen somit Verbindungen der Formel IV, worin R′ und R″ Phenylreste bedeuten. Die Abspaltung der Phenylgruppen kann mit Wasserstoff in Gegenwart von Rhodium/Kohle erfolgen. Analog kann man 1-Naphtholphthalein mit Diphenylphosphorylchlorid umsetzen und die Schutzgruppen durch Hydrierung in Gegenwart eines Rh/C-Katalysators entfernern. In jedem Fall werden Verbindungen der allgemeinen Formel I erhalten.

Ferner hat sich bei der Synthese von Monophosphaten der allgemeinen Formel I die Herstellung des Dibenzylphosphorsäureesters der allgemeinen Formel IV im wässrigen Medium durch Phasentransferkatalyse und anschließende katalytische Debenzylierung analog der Arbeiten von A. Zwierzak (Synthesis 1976, 305) bewährt. Als Phasentransferkatalysatoren können beispielsweise quartäre Ammoniumsalze wie Benzyltributyl-ammoniumbromid und Benzyltriethylammoniumbromid, Phosphoniumsalze wie z. B. Triphenylphosphoniumchlorid, Polyethylenglykole und/oder Kronenether und Gemische der hier beispielhaft genannten Verbindungen verwendet werden. Die Entfernung der nach säulenchromatographischer Reinigung anhaftenden Essigsäure wurde durch Lösen der Verbindung in Ammoniak und Auftragen auf eine HP-20-Absorbersäule (Polystyrol-divinylchlorbenzolharz) durchgeführt. Nach Elution mit Wasser, Isopropanol und ammoniakhaltigem Wasser erhält man vorwiegend reines Monophosphat der allgemeinen Formel I. Die Entfernung der Essigsäure ist auch durch Zugabe von Dicyclohexylamin in Methanol und nachfolgendem fraktionierten Fallen mit Ether möglich.

Die Bromierung von 1-Naphtholphthaleinmonophosphaten der allgemeinen Formel I, worin $R^1$ und $R^2$ Wasserstoff bedeuten und $M^1$ und $M^2$ die oben angegebene Bedeutung haben, in Ether gibt ein Gemisch der entsprechenden 2-Monobrom- und 2,2′-Dibromverbindung der allgemeinen Formel I, worin $R^1$ und $R^2$ Wasserstoff und/oder Brom bedeuten. Bevorzugt werden hierbei die monobromsubstituierten Verbindungen der Formeln I mit zur Phosphatgruppe orthoständigem Brom gebildet.

Unter "Halogen" in den Definitionen von $R^1$ bis $R^5$ ist Fluor, Chlor, Brom und Jod, vorzugsweise Chlor und Brom zu verstehen.

Unter "Alkalimetallkation" in der Definition von $M^1$ und $M^2$ ist das Lithium-, Natrium- und Kaliumion zu verstehen, wobei das Lithium- und Natriumion bevorzugt sind.

Das "Erdalkalimetallkation" in der Definition von $M^1$ und $M^2$ bedeutet Magnesium-, Calcium- und Bariumion, wobei das Calciumion bevorzugt ist.

Die "Alkylgruppierung" in dem "Tetraalkylammoniumkation" und die "Alkanolgruppierung" in dem "Alkanolammoniumkation" in der Definition von $M^1$ und $M^2$ enthalten 1 bis 5, vorzugsweise 1 oder 2 Kohlenstoffatome.

Unter der "Alkylarylgruppe" in der Definition von $R^3$ und $R^4$ sind Reste zu verstehen, die aus einer Kohlenstoffkette mit 1 bis 3, vorzugsweise 1 bis 2 Kohlenstoffatomen und einem aromatischen Rest, vorzugsweise dem Phenylrest, bestehen. Bevorzugt ist hier die Benzylgruppe.

Ein weiterer Gegenstand der Erfindung ist die Verwendung der neuen 1-Naphtholphthaleinmonophosphate der allgemeinen Formel I zur Bestimmung der Aktivität von alkalischen Phosphatasen.

Die Verwendung der 1-Naphtholphthaleinmonophosphate als Substrate für die alkalische Phosphatase führt zu deutlich empfindlicheren AP-Testsystemen, als sie bisher bekannt sind. Die neuen Substrate

können zur Bestimmung der Aktivität von alkalischen Phosphatasen mit Vorteil sowohl im biochemischen als auch im klinisch-chemischen Bereich eingesetzt werden.

Die neuen Monophosphatsubstrate verfügen aufgrund ihrer guten Löslichkeit in wässrigem Milieu im pH-Bereich von 10 bis 11 über hohe Reaktivitäten und über kurze lag-Phasen im photometrischen Phosphatase-Test. Damit sind folgende Vorteile verbunden:

a) Erniedrigung der Nachweisgrenzen,

b) kurze Reaktionszeiten und

c) geringerer Probeneinsatz und damit auch geringere Störungen durch andere Probenbestandteile.

Des weiteren zeigen die neuen Substrate wegen der bei den freigesetzten Anionen auftretenden großen Stokes-Verschiebung gegenüber der Absorption der Substrate nur eine geringfügige Überlappung mit der photometrisch zu verfolgenden Zunahme der Absorption des bei der enzymatischen Hydrolyse entstehenden Anions.

Die Bildung der rotviolett bis blau gefärbten Anionen (λ max 650 nm) erfolgt überraschenderweise bereits unter schwach alkalischen Bedingungen (z.B. bei pH 8). Hierdurch ist es erstmalig möglich, die Aktivität von alkalischen Phosphatasen photometrisch im tiefen sichtbaren Spektralbereich (650 - 670 nm) in kontinuierlicher Messung im schwach alkalischen pH-Bereich mit einer so hohen Nachweisempfindlichkeit zu bestimmen, wie es bisher nur mit zeitaufwendigeren nichtkontinuierlichen Verfahren möglich ist, und zugleich Störungen durch Probenbestandteile wie Bilirubin und andere Hämolyseprodukte, die in diesem Wellenlängenbereich nicht absorbieren, und unlösliche Bestandteile, beispielsweise Trübungen, zu eliminieren.

Die erfindungsgemäßen 1-Naphtholphthaleinmonophosphate können als Enzymsubstrate zur Bestimmung von AP-Aktivitäten in den verschiedensten Körperflüssigkeiten (Proben) wie beispielsweise Blut, Serum, Plasma, Urin, Speichel und ihre Ultrafiltrate oder Gewebsextrakte verwendet werden.

Die Bestimmung der alkalischen Phosphatase mit den erfindungsgemäßen Substraten kann in einem Konzentrationsbereich von 1,0 bis 20,0 mM erfolgen. Zweckmäßigerweise wird im Rahmen des erfindungsgemäßen Verfahrens eine Konzentration von 7,0 - 8,0 mM an 1-Naphtholphthaleinmonophosphat der allgemeinen Formel I verwendet.

Geeignet sind die 1-Naphtholphthaleinmonophosphate der Formel I auch für immunologische Bestimmungsmethoden, bei denen alkalische Phosphatase als Indikator-Enzym verwendet wird, dessen Aktivität nach Durchführung der immunologischen Reaktion ermittelt werden muß. Solche immunologische Bestimmungsmethoden mit enzymatischer Indikatorreaktion sind dem Fachmann als Enzymimmunoassays bekannt. Diese Methoden dienen zur Bestimmung der Konzentration von Proteinen, Polysacchariden, Hormonen, Arzneistoffen und anderen niedermolekularen Substanzen im Bereich von $10^{-5}$ bis $10^{-12}$ mol/l. Je nach Erfordernis von Phasentrennschritten unterscheidet man zwischen homogener und heterogener Testführung. Eine weitere Unterteilung kann in kompetitive und nicht-kompetitive Testprinzipien erfolgen.

Alle Testprinzipien arbeiten jedoch mit Enzym-Antigen- bzw. Enzym-Antikörper-Konjugaten. Die enzymatische Indikatorreaktion ist allen Enzymimmunoassays gemeinsam.

Ein für solche Zwecke geeignetes Indikatorenzym ist die alkalische Phosphatase. Die Bestimmung der alkalischen Phosphatase in solchen Enzymimmunoassays erfolgt üblicherweise, indem ein geeignetes AP-Substrat zugesetzt wird, das enzymatisch gespalten und in üblicher Weise photometrisch vermessen wird.

Eine Verbesserung des AP-Testsystems führt demnach ebenfalls zu erheblichen Vorteilen bei solchen Enzymimmunoassays:

1. Die höhere Empfindlichkeit ermöglicht auch hier eine weitere Erniedrigung der Nachweisgrenzen, kurze Reaktionszeiten und geringeren Probeneinsatz und damit auch geringere Störungen durch andere Probenbestandteile.

2. Die günstigere Meßwellenlänge vermindert bei bestimmten Reaktionsführungen die Störanfälligkeit der Methode durch unlösliche Bestandteile, beispielsweise durch Trübungen.

Ein weiterer Gegenstand der Erfindung sind diagnostische Mittel zur Bestimmung der Aktivität von alkalischen Phosphatasen, die die neuen 1-Naphtholphthaleinmonophosphate der allgemeinen Formel I enthalten.

Das diagnostische Mittel enthält neben einem oder mehreren der erfindungsgemäßen Substrate der allgemeinen Formel I, ein geeignetes Puffersystem, bestehend aus einem Aminoalkohol, wie z. B. N-Methyl-D-Glucamin/HCl, Diethanolamin/HCl, Triethanolamin/HCl und/oder 2-Amino-2-methyl-propanol(1)/HCl und gegebenenfalls weitere Substanzen mit Puffereigenschaften in dem für die AP-Bestimmung erforderlichen pH-Bereich, sowie gegebenenfalls weitere geeignete, üblicherweise für solche diagnostische Mittel verwendete Zusatzstoffe, wie beispielsweise Netzmittel, Stabilisatoren usw. Das diagnostische Mittel kann in Form einer Lösung, gegebenenfalls auf den gewünschten pH-Wert gepuffert, als Lyophilisat, als Pulvergemisch, Reagenztablette oder auf einem saugfähigen Träger aufgezogen oder in einem offenen Film vorliegen.

Das erfindungsgemäße diagnostische Mittel in Form einer Lösung enthält vorzugsweise sämtliche für den Test benötigten Reagentien. Als Lösungsmittel kommen Wasser oder Gemische von Wasser mit einem wasserlöslichen organischen Lösungsmittel, wie z. B. Methanol, Ethanol, Aceton oder Dimethylformamid, Dioxan, Glykol, in Frage. Aus Haltbarkeitsgründen kann es vorteilhaft sein, die für den Test benötigten Reagentien auf zwei oder mehr Lösungen zu verteilen, die erst bei der eigentlichen Untersuchung vermischt werden.

Zur Herstellung des diagnostischen Mittels in Form eines Lyophilisats im Gesamtgewicht von jeweils etwa 5 - 20 mg, vorzugsweise etwa 10 mg, wird eine Lösung getrocknet, die neben sämtlichen für den Test benötigten Reagentien übliche Gerüstbildner, wie z. B. Polyvinylpyrrolidon, und eventuell weitere Füllstoffe, wie z. B. Mannit, Sorbit oder Xylit enthält.

Ein diagnostisches Mittel in Form von Pulvermischungen oder Reagenztabletten läßt sich herstellen, indem die Bestandteile des Testes mit üblichen galenischen Zusatzstoffen versetzt und granuliert werden. Zusatzstoffe dieser Art sind z. B. Kohlenhydrate, wie z. B. Mono-, Oligo- oder Polysaccharide, oder Zuckeralkohole, wie z. B. Mannit, Sorbit oder Xylit, oder andere lösliche inerte Verbindungen, wie Polyethylenglykole oder Polyvinylpyrrolidon. Die Pulvermischungen oder Reagenztabletten weisen im allgemeinen ein Endgewicht von ungefährt 30 bis 200 mg, vorzugsweise 50 bis 80 mg, auf.

Zur Herstellung des diagnostischen Mittels in Form eines Teststreifens wird ein saugfähiger Träger, vorzugsweise Filterpapier, Cellulose oder Kunstfaservlies mit Lösungen der erforderlichen, überlicherweise zur Herstellung von Teststreifen verwendeten Reagentien in leicht flüchtigen Lösungsmitteln, wie z. B. Wasser, Methanol, Ethanol oder Aceton imprägniert. Dies kann in einem Imprägnierungsschritt erfolgen. Oft ist es jedoch zweckmäßig, die Imprägnierung in mehreren Schritten durchzuführen, wobei Lösungen eingesetzt werden, die jeweils einen Teil der Bestandteile des diagnostischen Mittels enthalten. So kann beispielsweise in einem ersten Schritt mit einer wäßrigen Lösung, die den Puffer und andere wasserlösliche Zusatzstoffe enthält, und dann in einem zweiten Schritt mit einer Lösung, die die erfindungsgemäßen alkalische Phosphatase-Substrate in einer Konzentration von 5 bis 20 mMol/l, vorzugsweise in einer Konzentration von 7 bis 8 mMol/l enthält, imprägniert werden.

Zur Herstellung des diagnostischen Mittels in Form eines Teststreifens kann ferner ein offener Film dienen, in dem neben Filmbildner und Pigmenten Substrat, Puffer und sonstige üblicherweise für diagnostische Mittel verwendete Zusatzstoffe enthalten sind. Die Naphtholphthaleinmonophosphate der allgemeinen Formel I werden hierbei in einem Konzentrationsbereich von 2 bis 20 mM, vorzugsweise in einer Konzentration von 7,5 bis 11,3 mM verwendet.

Die fertigen Testpapiere und Testfilme können als solche verwendet werden oder in an sich bekannter Weise an Trägerfolien angeklebt oder vorzugsweise zwischen Kunststoffen und feinmaschigen Netzwerken gemäß DE-PS 2118455 eingesiegelt werden und mit der zu untersuchenden Körperflüssigkeit (z.B. Blut, Plasma, Serum) in Verbindung gebracht werden.

Die nachstehenden Beispiele zeigen einige der zahlreichen Verfahrensvarianten, die zur Synthese der erfindungsgemäßen Verbindungen beschritten werden können sowie beispielhaft die Verwendung der neuen 1-Naphtholphthaleinmonophosphate zur Bestimmung der Aktivität von alkalischer Phosphatase. Sie sollen jedoch nicht eine Einschränkung des Erfindungsgegenstandes darstellen.

Die Herstellung der nachfolgend formelmäßig aufgeführten Substrate und Zwischenprodukte sind in den anschließenden Beispielen näher erläutert:

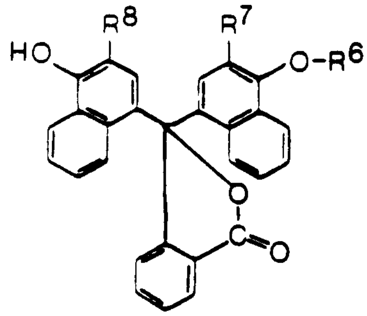

(V)

| | | | |
|---|---|---|---|
| (1) | $R^6 = PO_3H_2$; | $R^7 = R^8 = H$; | MG 498.5 |
| (2) | $R^6 = PO_3H_2$; | $R^7 = Br, R^8 = H$ | MG 577.3 |
| (3) | $R^6 = PO_3H_2$; | $R^7 = R^8 = Br$; | MG 656.2 |
| (4) | $R^6 = H$; | $R^7 = Br, R^8 = H$ | MG 497.4 |
| (5) | $R^6 = H$; | $R^7 = R^8 = Br$; | MG 576.3 |

**Beispiel 1**

1-Naphtholphthaleinmonophosphat (1) Phosphoroxichlorid-Methode

6.27 g (0.015 Mol) 1-Naphtholphthalein werden in eine Mischung von 25 ml absolutem Tetrahydrofuran und 1 ml (0.014 Mol) trockenem Pyridin eingetragen, unter Rühren mit 1.5 ml (0.015 Mol) Phosphoroxichlorid versetzt und 1 Stunde unter Rückfluß erhitzt. Danach gibt man zur Reaktionsmischung 100 ml 2 N Salzsäure und erwärmt 15 Minuten auf 70 °C, wobei sich ein dunkles Öl abscheidet. Dieses wird abgetrennt und zweimal mit je 50 ml 2 N Salzsäure wiederum 10 Minuten auf 70 °C erwärmt, abgetrennt, in 150 ml Wasser eingetropft und durch Zugabe von konzentrierter Natronlauge auf pH 10.3 gestellt. Die tiefgrüne Lösung wird einmal mit 125 ml
n-Butanol/Ligroin = 1/1, einmal mit 100 ml
n-Butanol/Ligroin = 1/3 und einmal mit 100 ml Ligroin geschüttelt. Danach wird die wässrige Phase mit konzentrierter Salzsäure auf pH 7.3 eingestellt und dreimal mit je 100 ml Essigester extrahiert. Die wässrige Phase wird am Rotationsdampfer zur Trockene eingeengt, wobei man 2 g braunes, amorphes Monophosphat erhält. Das Rohprodukt wird an einer Kieselgel-60-Säule (Höhe 80 cm, $\phi$ 4.5 cm) mit einer Mischung von Chloroform/Methanol/Methylethylketon/Essigsäure/Wasser = 75/35/25/5/9 getrennt.
Man erhält 1.05 g bräunliches, amorphes 1-Naphtholphthaleinmonophosphat.
Ausbeute: 14,1 % d. Th.
Fp. 226 °C.
Rf = 0.43 m vorgenannten Laufmittel.
(MG 498.5)

**Beispiel 2**

1-Naphtholphthaleinmonophosphat (1) Dibenzylphosphitmethode

83.6 g (0.2 Mol) 1-Naphtholphthalein werden in 840 ml absolutem Tetrahydrofuran gelöst, bei Raumtemperatur 40 ml (0.4 Mol) trockenes Tetrachlormethan zugegeben, danach innerhalb 20 Minuten 39.6 ml (0.18 Mol) Dibenzylphosphit und 82.8 ml (0.6 Mol) trockenes Triethylamin zugetropft, wobei die Temperatur auf 41 °C steigt. Man rührt 17 Stunden bei Raumtemperatur, wobei sich Triethylammoniumchlorid abscheidet. Das ungelöste Material wird über einen Faltenfilter abgetrennt und das Filtrat am Rotationsverdampfer eingeengt. Der Rückstand, 159 g dunkles Öl, wird in 400 ml Chloroform/Methanol = 9/1 gelöst und an einer Kieselgel-60-säule (Höhe 120 cm, $\phi$ 7.5 cm) gereinigt. Man erhält beim Einengen der betreffenden Fraktionen 72.4 g schwach verunreinigtes dunkles Öl, das durch erneutes Aufgeben auf eine Kieselgel-60-säule (Höhe 120 cm, $\phi$ 7.5 cm) mit Laufmittel Chloroform/Methanol = 29/1 DC-einheitlich erhalten wird.

Der so gereinigte Dibenzylester, 38.2 g amorpher, roter Schaum ($R_f$ = 0.4; Laufmittel Chloroform/Methanol = 29/1), wird in einer Mischung von 500 ml Methanol/Dioxan = 1/1 unter Zugabe von 1 g Palladiumoxid 4.5 Stunden bei 5 Atm. und 25 °C hydriert. Nach Absaugen vom Katalysator wird die gelbe Lösung am Rotationsverdamper zur Trockene eingeengt. Man erhält 26.7 g orangefarbenes, amorphes Produkt. Dieses wird an einer Kieselgel-60-säule (Höhe 120 cm, $\phi$ 7.5 cm, Laufmittel Chloroform/Methanol/Methylethylketon/Essigsäure/Wasser = 75/35/25/5/9) gereinigt. Das Rohprodukt, 19.6 g hellorangefarbenes Pulver wird mit 300 ml Ether angerieben, die gebildeten Kristalle abgesaugt, mit Ether gewaschen und 48 Stunden bei 50 °C über Diphosphorpentoxid und Kaliumhydroxid bis zur Gewichtskonstanz getrocknet.
Man erhält 17.3 g 1-Naphtholphthaleinmonophosphat.
Ausbeute: 17.4 % d. Th.

Fp. > 250 ° C.

Zur Entfernung anhaftender Essigsäure löst man das Produkt in 350 ml konzentriertem Ammoniak, vermischt mit 20 g HP-20-Absorberharz (Polystyrol-Divinylchlorbenzoharz) und engt am Rotationsverdampfer ein. Der Rückstand wird auf eine 1 l HP-20-Säule gegeben und danach mit 5 l Wasser, 7.5 l 10 % Isopropanol und schließlich mit 10 % ammoniakhaltigem Wasser eluiert. Die wässrig ammoniakalische Lösung wird im Vakuum eingeengt und der Rückstand bis zur Gewichtskonstanz getrocknet.
Man erhält 10.5 g reines 1-Naphtholphthaleinmonophosphat.
Ausbeute: 10,7 % d. Th.
Fp. > 250 ° C
$R_f$ = 0.48 (Laufmittel Isopropanol/n-Butylacetat/Wasser = 50/30/20)
$R_f$ = 0.22 (Laufmittel Chloroform/Methanol/Methylethylketon/Essigester/Wasser = 75/35/25/5/9

**Beispiel 3**

1-Naphtholphthaleinmonophosphat (1) Dibenzylphosphit-Phasentransferkatalyse

In einem 500 ml Dreihalskolben mit Rührer, Thermometer und Tropftrichter gibt man zu einer Lösung von 11 ml (0.05 Mol) Dibenzylphosphit in 100 ml trockenem Chloroform und 17 ml Tetrachlormethan, 2.6 ml (0.02 Mol) Triethylamin, rührt 1 Stunde bei Raumtemperatur, gibt dann 20.8 g (0.05 Mol) 1-Naphtholphthalein (90 % GC) und 3.23 g (0.01 Mol) Tetrabutylammoniumbromid zu und tropft bei 25 - 30 ° C eine Lösung von 55.3 g (0.4 Mol) Kaliumcarbonat in 100 ml Wasser während 10 Minuten unter Rühren zu. Nach einer Stunde Weiterrühren, wird die organische Phase abgetrennt, dreimal mit je 150 ml 1 N Natronlauge und dreimal mit je 150 ml Wasser extrahiert, über Natriumsulfat getrocknet und am Rotationsverdampfer eingeengt.
Man erhält 19.1 g braunes Öl.
$R_f$ = 0.4 (Laufmittel Chloroform/Methanol = 29/1, DC-Kieselgel 60-Merck)

Dieses Material wird in 300 ml Methanol/Dioxan = 1/1 gelöst und nach Zugabe von 1.5 g Pd/C 10 % hydriert. Nach 2 Stunden ist die Wasserstoffaufnahme beendet. Nach Absaugen des Katalysators wird die Reaktionslösung eingeengt. Man erhält 15.4 g rotes Harz. Dieses wird an einer Kieselgel-60-Säule (Höhe 105 cm, $\phi$ 5.5 cm) mit Chloroform/Methanol/Methylethylketon/Eisessig/Wasser = 75/35/25/5/9 gereinigt. Die DC-reinen Fraktionen werden eingeengt. Man erhält 7.3 g orangerotes Harz. Durch Zugabe von 100 ml Ether und Anreiben erhält man nach Absaugen, Waschen mit Ether und 24 Stunden Trocknen bei Raumtemperatur 6.4 g leicht orangefarbenes, amorphes Pulver.
Ausbeute: 25,6 % d. Th.
Fp. > 250 ° C
$R_f$ = 0.48 (Laufmittel Isopropanol/n-Butylacetat/Wasser = 50/30/20, DC HPTLC-Fertigplatte: Kieselgel 60 F 254 (Merck) )
$R_f$ = 0.32 (Laufmittel Chloroform/Methanol/Methylethylketon/Eisessig/Wasser = 75/35/25/5/9)

Das Produkt, hergestellt nach den Beispielen 1 bis 3, läßt sich mit AP in Gegenwart von Boratpuffer (pH 10.3) in 1-Naphtholphthalein ($\lambda$ max. = 650 nm, $\epsilon$ = 30 300 l/mol cm) überführen.

**Beispiel 4**

2-Monobrom-1-naphtholphthaleinmonophosphat (2)

Zu einer Suspension von 2 g (0.004 Mol) 1-Naphtholphthaleinmonophosphat in 50 ml Diethylether tropft man bei 0 ° C unter Rühren während 10 Minuten 0.4 ml (0.008 Mol) Brom in 10 ml Ether zu. Man rührt 1 Stunde bei 0-5 ° C nach, dekantiert den Ether von dem öligen Reaktionsprodukt ab und rührt den Rückstand mit 30 ml Ligroin an. Nach Absaugen und Trocknen über Molekularsieb erhält man 2 g beigefarbene 2-Monobrom-1-naphtholphthaleinmonophosphat.
Ausbeute: 86.6 % d. Th.
Fp. > 160 ° C (Zers.)
$R_f$ = 0.32 (Laufmittel Chloroform/Methanol/Methylethylketon/Wasser = 75/35/25/9, DC Kieselgelplatte 60-Merck)
(MG 577.4)

**Beispiel 5**

2,2'-Dibrom-1-naphtholphthaleinmonophosphat (3)

Man verfährt wie im vorangehenden Beispiel beschrieben und verwendet ein Molverhältnis 1-Naphtholphthaleinmonophosphat/Brom Brom = 1 : 3. Man erhält neben der bekannten Monobromverbindung das gewünschte 2,2,-Dibrom-1-naphtholphthaleinmonophosphat. Die Auftrennung erfolgt an einer Kieselgel 60-säule (Höhe 68 cm, $\phi$ 35 cm, Laufmittel Isopropanol/n-Butylacetat/Wasser Wasser = 50/30/20). Man erhält 0,61 g beigefarbenes, kristallines 2,2,-Dibrom-1-naphtholphthaleinmonophosphat.
Ausbeute: 23 % d. Th.
Fp. > 240 °C (Zers.)
$R_f$ = 0.25 (Laufmittel Chloroform/Methanol/Methylethylketon/Wasser = 75/35/25/9, DC Kieselgel 60-Merck)
(MG 656.2)

Die Verbindungen 2-Monobrom- und 2,2'-Dibrom-1-naphtholphthaleinmonophosphat, hergestellt nach den Beispielen 4 und 5, lassen sich in methanolischer Lösung in Gegenwart von Boratpuffer und/oder eines Aminoalkohols (pH 10.5) mit alkalischer Phosphatase spalten, wobei 2-Monobrom-1-naphtholphthalein ($\lambda$max. = 655 nm, $\epsilon$ = 25 300 l/Mol cm) und 2,2'-Dibrom-1-naptholphthalein ($\lambda$max. = 670 nm, $\epsilon$ = 27 800 l/Mol cm) entsteht.

**Beispiel 6**

2-Monobrom- und 2,2'-Dibrom-1-naphtholphthalein (4) und (5)

In einem 250 ml Dreihalskolben mit Rührer und Thermometer löst man 4.18 g (0.01 Mol) 1-Naphtholphthalein in 100 ml Diethylether und tropft bei 0 °C unter Rühren eine Lösung von 2.4 g (0.77 ml, 0.015 Mol) Brom in 80 ml Ether innerhalb von 2 Stunden zu. Nach Stehenlassen über Nacht, engt man im Vakuum ein und erhält 5.5 g eines Gemisches von etwa gleichen Teilen Mono- und Dibromverbindung. Die säulenchromatografische Auftrennung an Kieselgel 60-Merck erfolgt mit Toluol/Essigester = 15/1 oder Isopropanol/n-Butylacetat/Wasser = 50/30/20.
Man erhält 2,6 g beigefarbene, kristalline Dibromverbindung. Ausbeute: 45 % d. Th.
Fp. > 245 °C (Zers.)
$R_f$ = 0.5 (Laufmittel Toluol/Essigester = 5/1)
$\lambda$max = 670 nm
und 1,6 g beigefarbene, pulverartige Monobromverbindung.
Ausbeute: 30 % d. Th.
Fp > 240 °C (Zers.)
$R_f$ = 0.32 (Laufmittel Toluol/Essigester = 5/1)
$\lambda$max = 655 nm

**Beispiel 7**

Bestimmung der Aktivität der alkalischen Phosphatase durch Extinktionsphotometrie in der Küvette

a) Zubereitung der verwendeten Lösungen:

| | | |
|---|---|---|
| Pufferlösung: | N-Methyl-D-Glucamin<br>pH-Wert (mit 1 N Salzsäure eingestellt) | 1 mol/l<br>10,2 |

Reagenzlösung 1:

In der vorstehend beschriebenen Pufferlösung werden 7,5 mmol/l 1-Naphtholphthaleinmonophosphat-natriumsalz gelöst. Der pH-Wert wird kontrolliert (25 °C).

Reagenzlösung 2:

In der vorstehend beschriebenen Pufferlösung werden 7,5 mmol/l 2-Brom-1-naphtholphthaleinmonophosphatnatriumsalz gelöst. Der pH-Wert wird kontrolliert (25 ° C).

Reagenzlösung 3:

In der vorstehend beschriebenen Pufferlösung werden 7,5 mmol/l 2,2,-Dibrom-1-naphtholphthaleinmonophosphatnatriumsalz gelöst. Der pH-Wert wird kontrolliert (25 ° C).

Die Substratkonzentration und pH-Werte sind für jedes eingesetzte Substrat zu optimieren. Es können daher ganz bewußt unterschiedliche Werte für Substratkonzentrationen bzw. pH-Werte bei den einzelnen Reagenzlösungen auftreten. Die fertige Reagenzlösung ist bei Raumtemperatur 8 Stunden und bei 37 ° C 1,5 Stunden stabil.

Enzymlösung

Handelsübliche alkalische Phosphatase aus Kälberdarm wird in der vorstehend genannten Pufferlösung gelöst. Die Aktivität dieser Lösung beträgt ca. 3000 U/ml (bezogen auf die Herstellerdaten).

Probenlösung

Die Probe (z. B. zentrifugiertes Blut, Plasma, Serum) wird - falls erforderlich - mit der Pufferlösung verdünnt.

b) Durchführung der Messung

Die Messung erfolgt photometrisch bei der jeweils angegebenen Wellenlänge:

3 ml Reagenz werden in einer 1 cm-Küvette bei 37 ° C mit 50 ul der entsprechend verdünnten Probenlösung oder 50 ul Enzymlösung vermischt. Aus der Extinktionsänderung pro Zeiteinheit in (Δ E/min) und der gewählten Verdünnung wird die Aktivität der Enzymlösung bzw. Probe in bekannter Weise berechnet. Für eine resultierende Aktivität in der Küvette von 50 U/l wird ein Δ E/min von ca. 220 mE gefunden.

| Reagenz-Nr. | Meßwellenlänge (nm) |
|---|---|
| 1 | 650 |
| 2 | 655 |
| 3 | 670 |

**Beispiel 8**

Bestimmung der Aktivität der alkalischen Phosphatase durch Reflexionsphotometrie von Teststreifen

a) Herstellung von Teststreifen mit saugfähigem Reagenzträger

Tränklösung 1:

| | |
|---|---|
| N-Methyl-D-Glucamin | 2 mol/l |
| $MgCl_2$ | 1 mmol/l |
| $ZnCl_2$ | 15 umol/l |
| 1-Naphtholphthaleinmonophosphat | 7,5 mmol/l |
| pH-Wert (mit 1 N Salzsäure eingestellt) | 10,2 (25°C) |

Tränklösung 2:

| | |
|---|---|
| N-Methyl-D-Glucamin | 2 mol/l |
| $MgCl_2$ | 1 mmol/l |
| $ZnCl_2$ | 15 umol/l |
| 2-Brom-1-naphtholphthaleinmonophosphat | 7,5 mmol/l |
| pH-Wert (mit 1 N Salzsäure eingestellt) | 10,2 (25°C) |

Tränklösung 3:

| | |
|---|---|
| N-Methyl-D-Glucamin | 2 mol/l |
| $MgCl_2$ | 1 mmol/l |
| ZnCl2 | 15 umol/l |
| 2,2'-Dibrom-1-naphtholphthaleinmonophosphat | 7,5 mmol/l |
| pH-Wert (mit 1 N Salzsäure eingestellt) | 10,2 (25°C) |

Ein saugfähiger Träger (Papier, Vlies, Kunststoffgewebe) wird in bekannter Weise mit einer Tränklösung imprägniert. Der so erhaltene saugfähige Reagenzträger wird in bekannter Weise (DE-PS 2436598) auf einen Kunststoffstreifen befestigt, der in Verbindung mit einem Reflexionsphotometer (z. B. Reflotron) die Bestimmung der AP-Aktivität gestattet.

b) Herstellung von Teststreifen mit saugfähigem Reagenzfilm

| | | |
|---|---|---|
| Pufferlösung | N-Methyl-D-glucamin<br>pH-Wert | 2 mol/l<br>11,8 |

Filmbeschichtungsmischung

Die Pufferlösung wird im Verhältnis 1 : 1 mit einer 10prozentigen (v/v) wässrigen Lösung von Polyvinylalkohol (Mowiol 26/88) vermischt. Anschließend wird der pH-Wert mit 1 N Salzsäure auf 10,5 eingestellt.

Filmbeschichtungsmasse 1

Zu 500 g der Filmbeschichtungsmischung werden unter Rühren 100 g Kieselerde (Celaton MW 25) und 3 g 1-Naphtholphthaleinmonophosphat eingetragen.

Filmbeschichtungsmasse 2

Zu 500 g der Filmbeschichtungsmischung werden unter Rühren 100 g Kieselerde (Celaton MW 25) und 3,5 g 2-Brom-1-naphtholphthaleinmonophosphat eingetragen.

Filmbeschichtungsmasse 3

Zu 500 g der Filmbeschichtungsmischung werden unter Rühren 100 g Kieselerde (Celaton MW 25) und 4 g 2,2'-Dibrom-1-naphtholphthaleinmonophosphat eingetragen.

Die einzelnen luftblasenfreien Beschichtungsmassen werden in einer Naßdicke von 200 bis 500 um, vorzugsweise 300 um, beispielsweise mittels eines Rakels auf die Kunststoffolie aufgebracht. Anschließend wird 1/2 Stunde bei 60 ° C in einem Umlufttrockenschrank getrocknet.

Der so erhaltene Reagenzfilmträger wird in bekannter Weise (DE-PS 3130749) auf einem Kunststoffstreifen befestigt, der wie bei a) die Bestimmung der AP-Aktivität gestattet.

c) Durchführung der Messung

Die nach a) und b) hergestellten Teststreifen können in einem geeigneten Reflexionsphotometer, z. B. Reflotron, vermessen werden. Hier wird die Reaktion in bekannter Weise durch Kontaktschluß zwischen Probe und Papier-bzw. Filmreagenzträger gestartet. Die AP-Aktivität in der Probe wird aus der zeitlichen Änderung der Remission bei der jeweils angegebenen Wellenlänge bestimmt:

| Tränklösung Nr. | Filmbeschichtungsmasse Nr. | Meßwellenlänge (nm) |
|---|---|---|
| 1 | 1 | 650 |
| 2 | 2 | 655 |
| 3 | 3 | 670 |

## Patentansprüche

**1.** 1-Naphtholphthaleinmonophosphate der allgemeinen Formel I

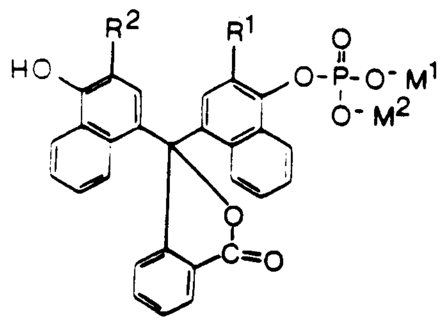

(I)

in der
$R^1$ und $R^2$, die gleich oder verschieden sein können, jeweils Wasserstoff oder Halogen
$M^1$ und $M^2$, die gleich oder verschieden sein können, jeweils ein Proton, ein Alkalimetall-, ein Erdkalimetall-, ein Ammonium-, ein Dicyclohexylammonium-, ein Tetraalkylammonium- oder ein Alkanolammoniumkation bedeuten,
und ihre tautomeren Umwandlungsprodukte.

**2.** 1-Naphtholphthaleinmonophosphat gemäß Anspruch 1, dadurch gekennzeichnet, daß $R^1$ und $R^2$ jeweils Wasserstoff und $M^1$ und $M^2$ jeweils ein Proton bedeuten.

**3.** 1-Naphtholphthaleinmonophosphat gemäß Anspruch 1, dadurch gekennzeichnet, daß $R^1$ Brom, $R^2$ Wasserstoff und $M^1$ und $M^2$ jeweils ein Proton bedeuten.

**4.** 1-Naphtholphthaleinmonophosphat gemäß Anspruch 1, dadurch gekennzeichnet, daß $R^1$ und $R^2$ jeweils Brom und $M^1$ und $M^2$ jeweils ein Proton bedeuten.

**5.** Verfahren zur Herstellung der 1-Naphtholphthaleinmonophosphate der allgemeinen Formel I

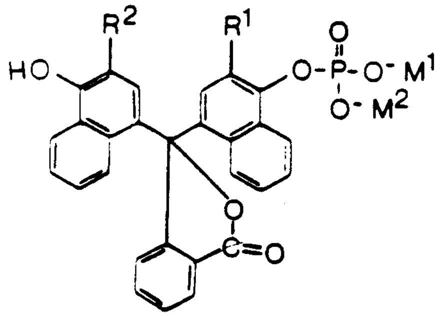

(I)

in der
$R^1$ und $R^2$, die gleich oder verschieden sein können, jeweils Wasserstoff oder Halogen
$M^1$ und $M^2$, die gleich oder verschieden sein können, jeweils ein Proton, ein Alkalimetall-, ein Erdkalimetall-, ein Ammonium-, ein Dicyclohexylammonium-, ein Tetraalkylammonium- oder ein Alkanolammoniumkation bedeuten, dadurch gekennzeichnet, daß man in an sich bekannter Weise Verbindungen der allgemeinen Formel II

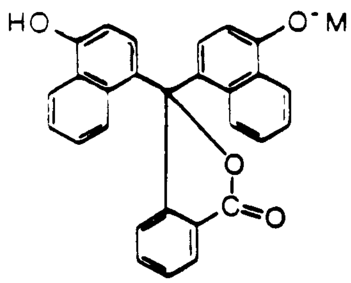

(II)

in der M ein Proton, ein Alkalimetall-, ein Erdkalimetall-, ein Ammonium-, ein Dicyclohexylammonium-, ein Tetraalkylammonium- oder ein Alkanolammoniumkation bedeutet, mit Phosphorverbindungen der allgemeinen Formel III

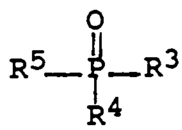

(III)

in der $R^3$ und $R^4$, die gleich oder verschieden sein können, jeweils Halogen, eine Aryl- oder eine Alkylarylgruppe und $R^5$ Wasserstoff oder Halogen bedeuten,
in Gegenwart einer organischen Base in wasserfreiem Lösungsmittel bzw. Lösungsmittelgemisch umsetzt und gegebenenfalls bromiert.

6. Verfahren nach Anspruch 5, dadurch gekennzeichnet, daß man 1-Naphtholphthalein mit Phosphorverbindungen der allgemeinen Formel III in Gegenwart einer organischen Base in wasserfreiem Lösungsmittel umsetzt und gegebenenfalls bromiert.

7. Verfahren nach Anspruch 5 oder 6, dadurch gekennzeichnet, daß als Phosphorverbindung Phosphoroxichlorid, Dibenzylphosphit, Diphenylphosphorylchlorid oder Diphenylphosphit verwendet wird.

8. Verfahren nach Anspruch 5 oder 6, dadurch gekennzeichnet, daß als organische Base Pyridin, Triethylamin und/oder entsprechende Homologe verwendet werden.

9. Verfahren nach einem der Ansprüch 5, 6, 7 und 8, dadurch gekennzeichnet, daß zusätzlich ein Phasentransferkatalysator zugefügt wird.

10. Verfahren nach Anspruch 9, dadurch gekennzeichnet, daß als Phasentransferkatalysator ein quartäres Ammoniumsalz verwendet wird.

11. Verwendung der 1-Naphtholphthaleinmonophosphate gemäß Anspruch 1 zur Bestimmung der Aktivität von alkalischer Phosphatase.

12. Diagnostisches Mittel zum Nachweis der alkalischen Phosphatase, enthaltend ein oder mehrere chromogene Substrate, eine geeignete Puffersubstanz sowie gegebenenfalls weitere üblicherweise verwendete Hilfsstoffe, dadurch gekennzeichnet, daß als chromogene Substrate 1-Naphtholphthaleinmonophosphate gemäß Anspruch 1 eingesetzt werden.

13. Diagnostisches Mittel gemäß Anspruch 12, dadurch gekennzeichnet, daß als zusätzliche Hilfsmittel Netzmittel, Quellmittel, Filmbildner, Füllstoffe wie z. B. Gerüstbildner und Pigmente und/oder weitere

galenische Zusatzstoffe verwendet werden.

**Claims**

**1.** 1-Naphtholphthalein monophosphates of the general formula I

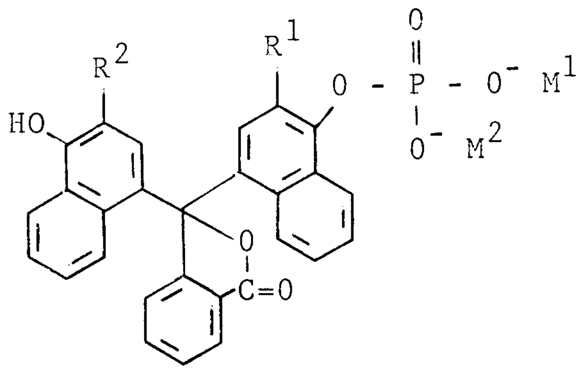

(I)

in which $R^1$ and $R^2$, which can be the same or different, each signify hydrogen or halogen, $M^1$ and $M^2$, which can be the same or different, each a proton, an alkali metal, an alkaline earth metal, an ammonium, a dicyclohexylammonium, a tetraalkylammonium or an alkanolammonium cation, and their tautomeric transformation products.

**2.** 1-Naphtholphthalein monophosphate according to claim 1, characterised in that $R^1$ and $R^2$ each signify hydrogen and $M^1$ and $M^2$ each a proton.

**3.** 1-Naphtholphthalein monophosphate according to claim 1, characterised in that $R^1$ signifies bromine, $R^2$ hydrogen and $M^1$ and $M^2$ each a proton.

**4.** 1-Naphtholphthalein monophosphate according to claim 1, characterised in that $R^1$ and $R^2$ each signify bromine and $M^1$ and $M^2$ each a proton.

**5.** Process for the preparation of the 1-naphtholphthalein monophosphates of the general formula I

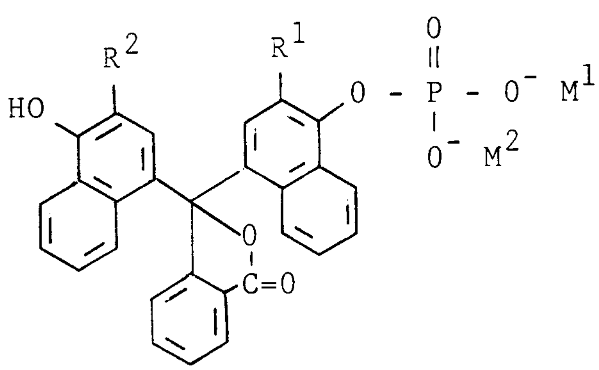

(I)

in which $R^1$ and $R^2$, which can be the same or different, each signify hydrogen or halogen, $M^1$ and $M^2$, which can be the same or different, each a proton, an alkali metal, an alkaline earth metal, an ammonium, a dicyclohexylammonium, a tetraalkylammonium or an alkanolammonium cation, characterised in that, in per se known manner, one reacts compounds of the general formula II

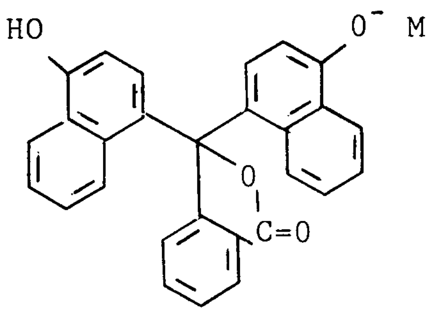

(II)

in which M signifies a proton, an alkali metal, an alkaline earth metal, an ammonium, a dicyclohexylammonium, a tetraalkylammonium or an alkanolammonium cation, with phosphorus compounds of the general formula III

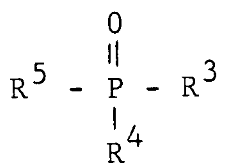

(III)

in which $R^3$ and $R^4$, which can be the same or different, each signify halogen, an aryl or an alkylaryl group and 35 hydrogen or halogen, in the presence of an organic base in anhydrous solvents or solvent mixtures and optionally brominates.

**6.** Process according to claim 5, characterised in that one reacts 1-naphtholphthalein with phosphorus compounds of the general formula III in the presence of an organic base in an anhydrous solvent and optionally brominates.

**7.** Process according to claim 5 or 6, characterised in that, as phosphorus compound, phosphorus oxychloride, dibenzyl phosphite, diphenylphosphoryl chloride or diphenylphosphite is used.

**8.** Process according to claim 5 or 6, characterised in that, as organic base, pyridine, triethylamine and/or corresponding homologues are used.

**9.** Process according to one of claims 5, 6, 7 and 8, characterised in that a phase transfer catalyst is additionally added thereto.

**10.** Process according to claim 9, characterised in that, as phase transfer catalyst, a quaternary ammonium salt is used.

**11.** Use of the 1-naphtholphthalein monophosphates according to claim 1 for the determination of the activity of alkaline phosphatase.

**12.** Diagnostic agent for the detection of alkaline phosphatase containing one or more chromogenic substrates, a suitable buffer substance, as well as possibly further adjuvants usually employed, characterised in that, as chromogenic substrate, 1-naphtholphthalein monophosphates according to claim 1 are used.

**13.** Diagnostic agent according to claim 12, characterised in that, as additional adjuvants, wetting agents, swelling agents, film formers, filler materials, such as e.g. structure formers and pigments, and/or further galenical additives are used.

## Revendications

1. Monophosphates de 1-naphtolphtaléine de formule générale I

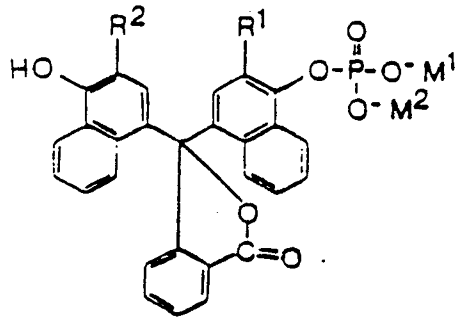

(I)

dans laquelle
$R^1$ et $R^2$, qui peuvent être identiques ou différents, peuvent représenter chacun un atome d'hydrogène ou d'halogène,
$M^1$ et $M^2$, qui peuvent être identiques ou différents, peuvent représenter chacun un proton, un cation de métal alcalin, un cation de métal alcalino-terreux, un cation d'ammonium, un cation de dicyclohexylammonium, un cation de tétraalkylammonium ou un cation d'alcanolammonium,
et leurs produits de transposition tautomères.

2. Monophosphates de 1-naphtolphtaléine selon la revendication 1, caractérisés en ce que $R^1$ et $R^2$ représentent chacun un atome d'hydrogène et $M^1$ et $M^2$ représentent chacun un proton.

3. Monophosphates de 1-naphtolphtaléine selon la revendication 1, caractérisés en ce que $R^1$ représente un atome de brome, $R^2$ représente un atome d'hydrogène et $M^1$ et $M^2$ représentent chacun un proton.

4. Monophosphates de 1-naphtolphtaléine selon la revendication 1, caractérisés en ce que $R^1$ et $R^2$ représentent chacun un atome de brome et $M^1$ et $M^2$ représentent chacun un proton.

5. Procédé de préparation des monophosphates de 1-naphtolphtaléine de formule générale I

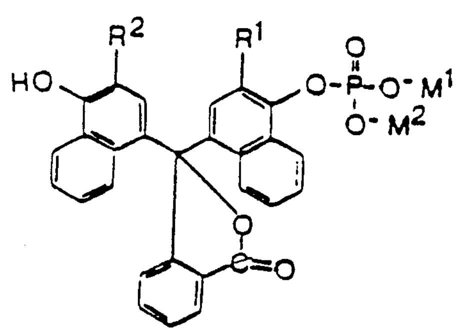

(I)

dans laquelle
$R^1$ et $R^2$, qui peuvent être identiques ou différents, peuvent représenter chacun un atome d'hydrogène ou d'halogène,
$M^1$ et $M^2$, qui peuvent être identiques ou différents, peuvent représenter chacun un proton, un cation de métal alcalin, un cation de métal alcalino-terreux, un cation d'ammonium, un cation de dicyclohexylammonium, un cation de tétraalkylammonium ou un cation d'alcanolammonium, caractérisé en ce que l'on fait réagir, de mamère connue en soi, des composés de formule générale II

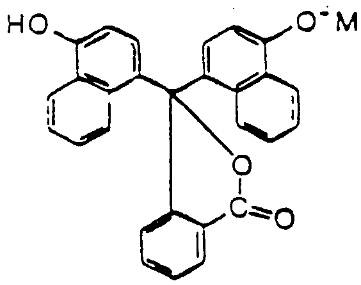

(II)

dans laquelle M représente un proton, un cation de métal alcalin, un cation de métal alcalino-terreux, un cation d'ammonium, un cation de dicyclohexylammonium, un cation de tétraalkylammonium ou un cation d'alcanolammonium, avec des composés du phosphore de formule générale III

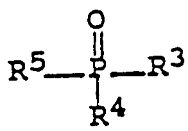

(III)

dans laquelle $R^3$ et $R^4$, qui peuvent être identiques ou différents, représentent chacun un atome d'halogène, un groupe aryle ou alkylaryle, et $R^5$ représente un atome d'hydrogène ou d'halogène, en présence d'une base organique, dans un solvant ou mélange de solvants anhydre, et éventuellement, on brome.

**6.** Procédé selon la revendication 5, caractérisé en ce que l'on fait réagir la 1-naphtolphtaléine avec des composés du phosphore, de formule générale III, en présence d'une base organique dans un solvant anhydre, et éventuellement on brome.

**7.** Procédé selon la revendication 5 ou 6, caractérisé en ce que comme composé du phosphore, on utilise l'oxychlorure de phosphore, le dibenzylphosphite, le chlorure de diphénylphosphoryle ou le diphénylphosphite.

**8.** Procédé selon la revendication 5 ou 6, caractérisé en ce que comme base organique, on utilise la pyridine, la triéthylamine et/ou leurs homologues appropriés.

**9.** Procédé selon l'une quelconque des revendications 5, 6, 7 et 8, caractérisé en ce que l'on ajoute en plus un catalyseur de transfert de phase.

**10.** Procédé selon la revendication 9, caractérisé en ce que comme catalyseur de transfert de phase, on utilise un sel d'ammonium quaternaire.

**11.** Utilisation des monophosphates de 1-naphtolphtaléine selon la revendication 1, pour la détermination de l'activité de la phosphatase alcaline.

**12.** Agent de diagnostic pour la détermination de la phosphatase alcaline, contenant un ou plusieurs substrats chromogènes, un tampon approprié ainsi qu'éventuellement d'autres adjuvants usuels, caractérisé en ce que comme substrat chromogène, on utilise des monophosphates de 1-naphtolphtaléine selon la revendication 1.

**13.** Agent de diagnostic selon la revendication 12, caractérisé en ce que comme adjuvant supplémentaire, on utilise un agent mouillant, un agent gonflant, un formateur de film, des charges, tels que par exemple des agents de structure et des pigments et/ou d'autres adjuvants galéniques.